# EUROPEAN PATENT APPLICATION

(11) **EP 4 495 598 A1**
(43) Date of publication of application: **22.01.2025**
(21) Application number: 22931279.8
(22) Date of filing: 14.03.2022
(51) Int. Cl.: G01N 33/68, G01N 33/574, G01N 15/10, G01N 21/64, G01N 1/34

(54) **CELL SORTING METHOD**

(71) Applicant: Tseng, Fan-Gang, Hsinchu Taiwan 30013 (TW)
(72) Inventor: WU, Jen-Kuei, Hsinchu, Taiwan 30013 (CN); CHIEN, Chih-Hsuan, Hsinchu, Taiwan 30013 (CN); TSENG, Fan-Gang, Hsinchu, Taiwan 30013 (CN)
(74) Representative: Becker, Eberhard
(86) International application number: PCT/CN2022/080546
(87) International publication number: WO 2023/173239

(57) **Abstract**

Provided is a cell sorting method capable of improving the accuracy and the capture purity of cell sorting. The method comprises the following steps: providing a blood sample; centrifuging the blood sample, so as to acquire a cell mixture which includes target cells and non-target cells; adding a fluorescent marker to the cell mixture, such that the fluorescent marker is combined with the target cells; adding an aqueous colloidal solution, such that the cell mixture is mixed with the aqueous colloidal solution to form a mixed solution; adding the mixed solution to a cell well of an array wafer, such that the target cells and the non-target cells in the cell mixture are laid on the bottom of the cell well in a roughly single layer manner; curing the mixed solution; and performing fluorescence image analysis, and selecting target cells which emit fluorescence after being combined with the fluorescent marker, so as to separate the selected target cells.

## Description

### BACKGROUND

### Technical Field

The disclosure relates to a cell sorting method, and more particularly, to a cell sorting method that screens and captures non-adhesive cells using an array chip and a hydrogel solution.

### Description of Related Art

Rare cells can be used in medical treatment, detection and analysis or other fields, such as cancer prognosis analysis, prenatal test or virus infection detection and analysis. Blood samples such as circulating tumor cells (CTCs), fetal nucleated red blood cells (FnRBCs), stem cells, etc. are typical rare cells. At present, rare cell detection and sorting systems are mostly used in the fields of circulating tumor cells (CTCs) and fetal nucleated red blood cells (FnRBCs). Since the number of such cells is extremely rare, a detection and sorting platform with high purity, high throughput, high cell recovery rate, high efficiency, and low cell damage is the goal of the current rare cell detection and sorting system.

### SUMMARY

The disclosure provides a cell sorting method capable of effectively improving the accuracy and capture purity of cell sorting while having the effect of low cell damage.

A cell sorting method of the disclosure includes following steps. A blood sample is provided. The blood sample is centrifuged to obtain a cell mixture having target cells and non-target cells. Fluorescent markers are added to the cell mixture to allow the fluorescent markers to bind with the target cells. A hydrogel solution is added and mixed with the cell mixture to form a mixture solution. The mixture solution is distributed to cell wells of an array chip, and the target cells and the non-target cells in the cell mixture are spread on a bottom of the cell wells in a substantially monolayer manner. The mixture solution is cured. A fluorescence image analysis is performed to select target cells that are bound with the fluorescent makers and emit fluorescence, and the selected target cells are isolated.

In an embodiment of the disclosure, the hydrogel solution includes a temperature-sensitive hydrogel solution, and a step of performing the fluorescence image analysis is performed after a step of curing the mixture solution. The step of curing the mixture solution includes: a cooling process is performed to cure the mixture solution, positions of the target cells and the non-target cells are fixed, and then the fluorescence image analysis is performed to locate the positions of the target cells that emit fluorescence so as to select the target cells that are bound with the fluorescent markers and emit fluorescence.

In an embodiment of the disclosure, taking a weight of the temperature-sensitive hydrogel solution as 100% by weight, the temperature-sensitive hydrogel solution includes 3% to 5% by weight of gelatin.

In an embodiment of the disclosure, the hydrogel solution includes a photosensitive hydrogel solution, and a step of performing the fluorescence image analysis is performed before a step of curing the mixture solution. The step of performing the fluorescence image analysis includes: the fluorescence image analysis is performed to locate a first region that emits fluorescence and a second region that does not emit fluorescence. The step of curing the mixture solution includes: the first region is irradiated with ultraviolet light to cure the mixture solution located in the first region, so as to fix the target cells.

In an embodiment of the disclosure, a step for isolating the selected target cells includes following. The uncured mixture solution located in the second region is removed after the first region is irradiated with ultraviolet light, and the fixed target cells are aspirated from the first region using an aspirator.

In an embodiment of the disclosure, the hydrogel solution includes a photosensitive hydrogel solution, and a step of performing the fluorescence image analysis is performed before a step of curing the mixture solution. The step of performing the fluorescence image analysis includes: the fluorescence image analysis is performed to locate a first region that emits fluorescence and a second region that does not emit fluorescence. The step of curing the mixture solution includes: the second region is irradiated with ultraviolet light to cure the mixture solution located in the second region.

In an embodiment of the disclosure, a step of isolating the selected target cells includes: the uncured mixture solution is aspirated from the first region by using an aspirator to obtain the target cells after the second region is irradiated with ultraviolet light.

In an embodiment of the disclosure, taking a weight of the photosensitive hydrogel solution as 100% by weight, the photosensitive hydrogel solution includes 10% to 20% by weight of polyethylene glycol diacrylate; and 0.1% to 1 wt% by weight of photoinitiator.

In an embodiment of the disclosure, the cells in the cell mixture are non-adhesive cells, including mononuclear lymphocytes, circulating tumor cells, fetal nucleated red blood cells, platelets, or a combination thereof.

In an embodiment of the disclosure, the target cells are fetal nucleated red blood cells. The fluorescent markers include an anti-CD147 antibody with a first fluorescent substance and an anti-CD71 antibody with a second fluorescent substance. A step of adding the fluorescent markers to the cell mixture includes: the anti-CD147 antibody and the anti-CD71 antibody are added to bind to allow the anti-CD147 and the anti-CD71 to bind with the target cells. The first fluorescent substance is different from the second fluorescent substance.

In an embodiment of the disclosure, the fluorescent markers further include 4',6-diamidino-2-phenylindole for binding with the target cells.

Based on the above, in the cell sorting method according to an embodiment of the disclosure, by spreading the cell mixture on the bottom of the cell wells in a substantially monolayer manner, the target cells and non-target cells will not overlap in the normal direction of the cell wells; next, by curing the mixture solution including the hydrogel solution, the target cells and/or non-target cells can be fixed on the bottom of the cell wells; then, by fluorescence image analysis, the target cells that are bound with the fluorescent markers and emit fluorescence can be located, and then the target cells can be precisely selected and isolated from the cell mixture. In this way, the cell sorting method of this embodiment may improve the accuracy and capture purity of cell sorting, so as to reduce cell damage and achieve the effect of high cell recovery rate.

### BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure.
Figure 1A to Figure 1D are schematic flowcharts of a cell sorting method according to an embodiment of the disclosure.
Figure 2A and Figure 2B are schematic diagrams of a self-assembled-cells-array chip according to an embodiment of the disclosure.
Figure 3A to Figure 3C are schematic flowcharts of a cell sorting method using a temperature-sensitive hydrogel solution according to an embodiment of the disclosure.
Figure 4A to Figure 4D are schematic flowcharts of a cell sorting method using a photosensitive hydrogel solution according to an embodiment of the disclosure.
Figure 5 is a schematic diagram of a photomask designed and used for irradiating the photosensitive hydrogel solution used in the cell sorting method according to an embodiment of the disclosure.
Figure 6A is a cell viability test result after a cell mixture is mixed with the temperature-sensitive hydrogel solution according to an embodiment of the disclosure.
Figure 6B is a cell viability test result obtained after extraction and isolation by the cell sorting method according to the embodiment of the disclosure.

Description of reference signs in the attached figures
10: plasma; 20: cell mixture; 25: mixed solution; 30: cell/ monocyte isolation solution; 40: red blood cell; 100: self-assembled-cells-array chip; 110: upper plate; 120: lower plate; 130: hole; 132: cell well; 134: evapotranspiration slot; 140: gap; S: blood sample; C1: target cells; C2: Non-target cells; PP: automatic aspirator; MEM: filter membrane; LL: Light.

### DESCRIPTION OF THE EMBODIMENTS

Reference will now be made in detail to the exemplary embodiments of the disclosure, examples of which are illustrated in the accompanying drawings. Wherever possible, the same reference numbers are used in the drawings and the description to refer to the same or like parts.

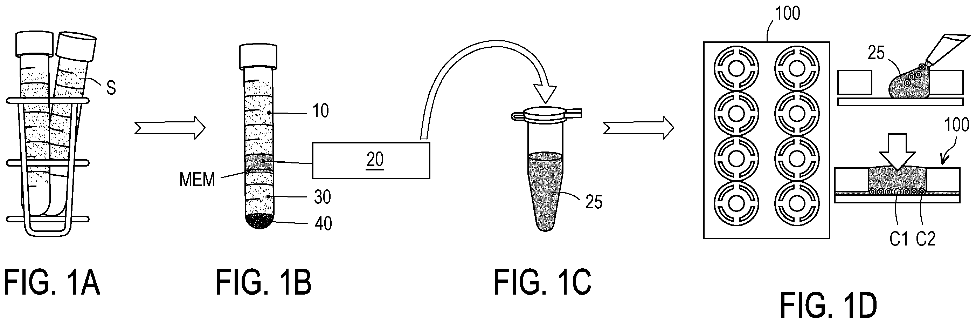

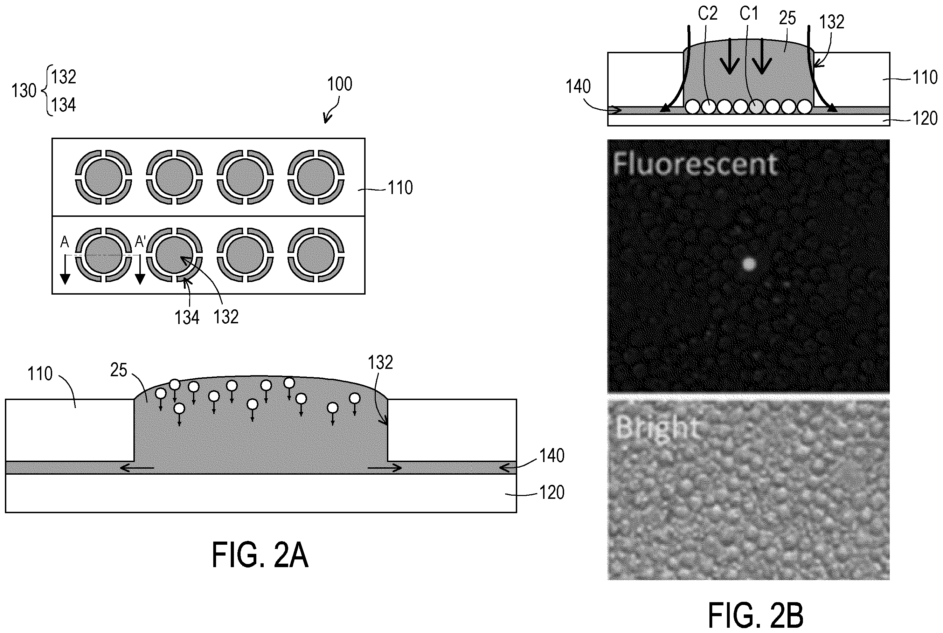

Figure 1A to Figure 1D are schematic flowcharts of a cell sorting method according to an embodiment of the disclosure. Figure 2A and Figure 2B are schematic diagrams of a self-assembled-cells-array chip according to an embodiment of the disclosure.

Referring to Figure 1A, a physiological sample (e.g. a blood sample S) is obtained from a test object to provide a test sample. For example, the test object is human or mammal, and the blood sample S is, for example, whole blood, but the disclosure is not limited thereto.

Next, referring to Figure 1B, the blood sample S provided is centrifuged to obtain a cell mixture 20, and the cell mixture 20 obtained by isolation has target cells C1 and non-target cells C2 that are to be isolated in the embodiment of the disclosure. The cells in the cell mixture 20 are mainly, for example, non-adhesive cells (i.e. suspended cells), and may include, for example, mononuclear lymphocytes, circulating tumor cells (CTCs), fetal nucleated red blood cells (FnRBCs), platelets, or a combination thereof. According to certain embodiments, the target cells C1 may include, for example, fetal nucleated red blood cells, and the non-target cells C2 may include, for example, mononuclear lymphocytes, circulating tumor cells, and/or platelets. According to some embodiments, the target cells C1 may include, for example, circulating tumor cells, and the non-target cells C2 may include, for example, mononuclear lymphocytes, fetal nucleated red blood cells, and/or platelets and so on, but the disclosure is not limited thereto.

To be specific, for example, Ficoll-PaqueTM cell/monocyte isolation solution (a solution with a density of 1.077 g/ml prepared from Ficoll and sodium diatrizoate in proportion) may be used to stratify blood components according to a density gradient, and the procedure is roughly as follows: firstly, LymphoprepTM is dropped into a Leucosep centrifuge tube below a filter membrane MEM. Next, the blood sample S is slowly poured along a wall of the centrifuge tube and centrifuged at 800×g (relative centrifugal force, RCF) for 15 minutes. At this time, the blood sample S will be stratified due to the centrifugation and the density of the LymphoprepTM solution, as shown in Figure 1B, sequentially red blood cells 40, a cell/monocyte isolation solution 30, the cell mixture 20, and the plasma 10 from bottom to top (i.e. from the bottom of the centrifuge tube to the top). In this embodiment, the cell mixture 20 may be located above the filter membrane MEM.

Next, referring to Figure 1C, the cell mixture 20 is extracted and isolated into a microcentrifuge tube, and fluorescent markers are added to the cell mixture 20 to allow the fluorescent markers to bind with the target cells C1 in the cell mixture 20. That is, a fluorescent staining is performed on the target cells C1. To be specific, the liquid (including the cell mixture 20 and the plasma 10) above the filter membrane MEM in the centrifuge tube is transferred to another brand new centrifuge tube and centrifuged at 300×g for 10 minutes to allow the cells in the cell mixture 20 to settle to the bottom of centrifuge tube. Next, after supernatant is removed and 1 milliliter (ml) of phosphate buffered saline (PBS) is added to allow the cells in the cell mixture 20 to resuspend, cell counts are performed on a portion of suspension (approximately 5 microliters (µl)) removed, and remaining suspension is centrifuged at 400×g for 6 minutes to allow the cells in the cell mixture 20 to settle to the bottom of the centrifuge tube. Next, a two-stage fluorescent staining is performed. The first stage of fluorescent staining is, for example, staining the surface antigens of cells, and the second stage of fluorescent staining is, for example, staining the nucleus of cells, wherein the exemplary steps are roughly as follows. Firstly, the supernatant is removed, and after 100 µl of PBS is added to the cells in the cell mixture 20 to resuspend, a first-stage fluorescent marker is added and light is avoided for 30 minutes, to allow the first-phase fluorescent marker to bind with specific cells in the cell mixture 20. Next, 1 ml of PBS is added and centrifuged at 400×g for 6 minutes, and then the supernatant is removed to remove the first-stage fluorescent marker that is not bound with surface antigens of the cells. Then, after 100 µl of PBS is added to allow the cells in the cell mixture 20 to resuspend, a second-stage fluorescent marker is added and light is avoided for 10 minutes to allow the second-stage fluorescent marker to bind with the nuclei of all cells in the cell mixture 20. Next, 1 ml of PBS is added and centrifuged at 400×g for 6 minutes, and then the supernatant is removed to remove the second-stage fluorescent marker that is not bound with cells.

For example, when the target cells C1 are fetal nucleated red blood cells, the first-stage fluorescent marker may include the anti-CD147 antibody with a first fluorescent substance and the anti-CD71 antibody with a second fluorescent substance, and the second-stage fluorescent marker may be, for example, 4',6-diamidino-2-phenylindole (DAPI). The first fluorescent substance is, for example, fluorescein isothiocyanate (FITC), with a fluorescence band of excitation wavelength Ex: 482 ± 25 nm/emission wavelength Em: 531 ± 40 nm, which emits green fluorescence when excited. The second fluorescent substance is, for example, phycoerythrin (PE), which has a fluorescence band of excitation wavelength Ex: 554±23 nm/emission wavelength Em: 624±40 nm, which emits orange fluorescence after being excited. DAPI has a fluorescence band of excitation wavelength Ex: 357±44 nm/emission wavelength Em: 475±28 nm, which emits blue fluorescence when excited. The anti-CD147 antibody and the anti-CD71 antibody bind with surface antigen CD147 and surface antigen CD71 of fetal nucleated red blood cells, respectively, while DPAI binds with the nucleus. The first fluorescent substance is different from the second fluorescent substance, and the first-stage fluorescent marker is also different from the second-stage fluorescent marker. In particular, each fluorescent marker has a different fluorescence emission wavelength range.

For example, when the target cells C1 are circulating tumor cells, the first-stage fluorescent marker may include an anti-EpCAM antibody with the first fluorescent substance, and the second-stage fluorescent marker may include Hoechst 33342. The first fluorescent substance is, for example, FITC having a fluorescence band of excitation wavelength Ex: 482 ± 25 nm/emission wavelength Em: 531 ± 40 nm, which emits green fluorescence when excited. The Hoechst 33342 has a fluorescence band of excitation wavelength Ex: 357±44 nm/emission wavelength Em: 475±28 nm, which emits blue fluorescence when excited. The anti-EpCAM antibody binds with surface antigen EpCAM of circulating tumor cells, and the Hoechst 33342 binds with the nucleus. Here, each fluorescent marker has a different fluorescence emission wavelength range.

It is particularly noted that, in some embodiments, the first stage of fluorescent staining may further include fluorescent staining the surface antigens of the non-target cells C2. For example, when the target cells C1 are circulating tumor cells, the first-stage fluorescent marker may include an anti-CD45 antibody with a second fluorescent substance, wherein the second fluorescent substance, for example, has a fluorescence band of excitation wavelength Ex: 554 ± 23 nm/emission wavelength Em: 624 ± 40 nm, which emits orange fluorescence when excited. The anti-CD45 antibody is the surface antigen CD71 that binds with leukocytes (i.e. the non-target cells C2), and may be used to mark the positions of leukocytes. Therefore, in the subsequent step of locating the positions of the target cells C1 by fluorescence image analysis, the region that emits orange fluorescence may be regarded as the excluded region. By this design, the positions of the target cells C1 can be more accurately located.

Next, referring to Figure 1C, Figure 1D, Figure 2A and Figure 2B at the same time, after fluorescent staining, a hydrogel solution is added to allow the cell mixture 20 to mix with the hydrogel solution to form a mixture solution 25. Then, the mixture solution 25 is distributed to cell wells 132 of an array chip 100 in a quantitative manner, such that the target cells C1 and the non-target cells C2 in the cell mixture 20 are spread on the bottom of the cell wells 132 in a substantially monolayer manner. That is, the target cells C1 and the non-target cells C2 do not overlap in the normal direction of the cell wells 132.

In the embodiment of the disclosure, the used hydrogel solution may be, for example, a temperature-sensitive hydrogel solution or a photosensitive hydrogel solution, but the disclosure is not limited thereto. The temperature-sensitive hydrogel solution is a hydrogel solution that cures or liquefies at a specific temperature. In this embodiment, taking a weight of the temperature-sensitive hydrogel solution as 100% by weight, the temperature-sensitive hydrogel solution is a hydrogel solution including 3% to 5% by weight of gelatin prepared by dissolving gelatin in water, but the disclosure is not limited thereto. Preferably, the temperature-sensitive hydrogel solution includes 3.5% by weight of gelatin. The temperature-sensitive hydrogel solution of this embodiment may be melted into a liquid state at a specific temperature (e.g. 37 °C or room temperature), and cured into a solid state at a low temperature (e.g. 4 °C).

The photosensitive hydrogel solution is a hydrogel solution that may be cured under light of a specific wavelength. In this embodiment, taking the weight of the photosensitive hydrogel solution as 100% by weight, the photosensitive hydrogel solution includes 10% to 20% by weight of polyethylene glycol diacrylate (pEGDA) with 0.1% to 1% by weight of a photoinitiator (lithium phenyl-2,4,6-trimethylbenzoylphosphinate; LAP), but the disclosure is not limited thereto. Preferably, the photosensitive hydrogel solution includes 15 wt % of polyethylene glycol diacrylate and 0.5 wt % of the lithium phenyl-2,4,6-trimethylbenzoylphosphinate. The photosensitive hydrogel solution of this embodiment may be cured into a solid state after being irradiated by UV light with a wavelength of 405 nanometers (nm) for about 15 seconds.

The array chip may be, for example, a self-assembled-cells-array chip (SACA Chip) 100, as shown in Figure 2A and Figure 2B. The self-assembled-cells-array chip 100 is formed by sandwiching an upper and a lower PC injection molded plastic plates 110 and 120. The upper plate 110 has eight groups of holes 130, the lower plate 120 is a flat surface having a hydrophilic and anti-cell adhesion coating, and there is a gap 140 of about 5 µm between the upper plate 110 and the lower plate 120. Each hole 130 is composed of one cell well 132 and four evapotranspiration slots 134, and the cell well 132 has a diameter of about 7 millimeters (mm).

To be specific, in this embodiment, a hydrogel solution (for example, a temperature-sensitive hydrogel solution or a photosensitive hydrogel solution) is first prepared, and an appropriate amount of hydrogel solution is added to allow the cells in the cell mixture 20 having fluorescent markers to resuspend, so as to form the mixture solution 25. Next, the mixture solution 25 is distributed to the cell wells 132. At this time the liquid will flow from the gap 140 to the evapotranspiration slots 134, and through structure-driven flow field and gravity field, the cells will settle down and align to the left and right sides. Since the gap 140 is smaller than the cell size, the cells do not flow out of the cell wells 132. As long as the cells in each cell well 132 do not exceed the limit (about 5 × 10⁵ cells), the cells may be spread into a single dense layer, as shown in Figure 2B, which can effectively improve the image recognition rate of subsequent cells, reduce the probability of misjudgment, and further improve the purity during sorting. In contrast, there is no gap between the upper and lower plates of a traditional orifice plate (for example, orifice plate of a 96-hole plate), so when the mixture solution 25 is distributed to the well, the liquid may only be stored in the well, and cell stacking is likely to occur. Cell stacking may cause difficulty in subsequent image recognition, making it difficult to select target cells, thereby reducing the accuracy and purity of cell sorting.

Next, the mixture solution 25 is cured, and the target cells C1 that are bound with the fluorescent markers and emit fluorescence are selected, and the selected target cells C1 are isolated. Note that in the disclosure, according to the type of hydrogel solution selected (temperature-sensitive hydrogel solution or photosensitive hydrogel solution), different curing methods are used, the sequence of subsequent steps such as mixture solution curing and cell selection will also be different, which will be described separately below.

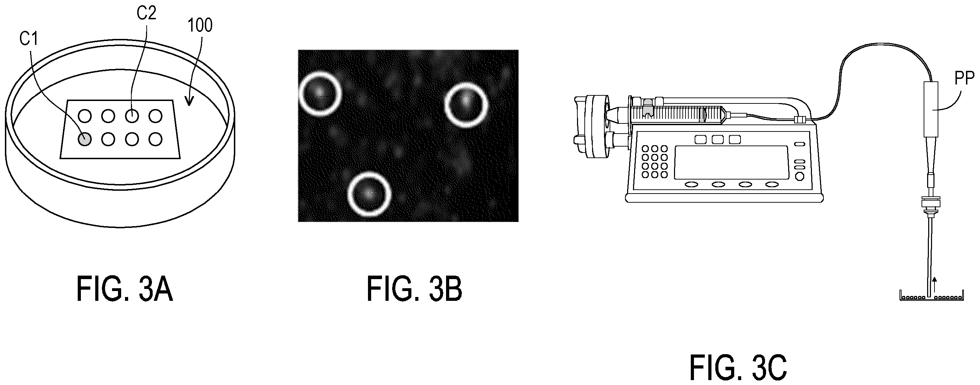

Continuing with the process of Figure 1A to Figure 1D, the processes shown in Figures 3A to 3C are schematic flowcharts of a cell sorting method using a temperature-sensitive hydrogel solution according to an embodiment of the disclosure.

Referring to Figure 1D and Figure 3A at the same time, the hydrogel solution used is a temperature-sensitive hydrogel solution, which is injected into the cell wells 132 of the array chip 100 in a liquid state, and the cells are spread in a substantially monolayer manner. Then, a cooling process is performed to cure the mixture solution 25 in the cell wells 132 to allow all the target cells C1 and all the non-target cells C2 in the mixture solution 25 in the cell wells 132 to be fixed, and the positions of all the target cells C1 and the non-target cells C2 in the cell wells are also fixed. In detail, the cooling process may be, for example, placing the mixture solution 25 in a 4° C environment (such as a refrigerator) for about 5 to 10 minutes, such that the mixture solution 25 is in a viscous state. The mixture solution 25 being in a viscous state indicates that the viscosity of the liquid increases and the fluidity decreases, and the target cells C1 and the non-target cells C2 in the mixture solution 25 are also fixed, which is beneficial to the subsequent precise locating and capturing of the positions of the target cells C1 and the non-target cells C2.

Next, referring to Figure 3B, a fluorescence image analysis is performed, and the positions of the target cells that emit fluorescence are located by automated image analysis. In detail, the array chip 100 including the mixture solution 25 is placed on an image analysis machine, and the fluorescence image analysis may be performed, for example, through a fluorescence image analysis system, for the array chip 100 including the mixture solution 25 on the machine, to calibrate the xyz axis, so as to confirm that the entire array chip 100 is in the correct position, thereby the relative positions of the cells and the cell wells of the array chip can be accurately located. Next, for the various fluorescent markers used in the examples, fluorescence scanning is performed on various specific fluorescence excitation bands (meaning the fluorescence excitation bands that correspond to specific fluorescent markers), and the images captured in different fluorescence bands are overlaid to further confirm the positions of the target cells C1. For example, when the target cells C1 are fetal nucleated red blood cells, and the fluorescent markers are the anti-CD147 antibody with a first fluorescent substance, the anti-CD71 antibody with a second fluorescent substance, and DAPI, the positions of the target cells C1 are the positions where all three, the fluorescence when the first fluorescent substance is excited, the fluorescence when the second fluorescent substance is excited, and the fluorescence when DAPI is excited, overlap at the same time (see selected circle as shown in Figure 3B). When the target cells C1 are circulating tumor cells, and the fluorescent markers are the anti-EpCAM antibody with the first fluorescent substance, the anti-CD45 antibody with the second fluorescent substance, and the Hoechst 33342, the positions of the target cells C1 are located in a region where the excited fluorescence of the first fluorescent substance overlaps with the excited fluorescence of the Hoechst 33342 and does not overlap with the excited fluorescence of the second fluorescent substance.

Next, referring to Figure 3C, the target cells C1 that are bound with the fluorescent markers and emit fluorescence are selected. In detail, the method of selecting the target cells C1 may be, for example, according to the image of the overlay, using an automatic aspirator PP of the computer-programmable electric single-cell extraction and injection system to obtain the target cells C1 by quantitative aspiration at a fixed flow rate (e.g. 20 microliters per minute (µl/min)), thereby realizing the function of single cell extraction and reducing background noise and contamination associated with the sorted solution at the same time. The cell sorting using the temperature-sensitive hydrogel solution is completed herein.

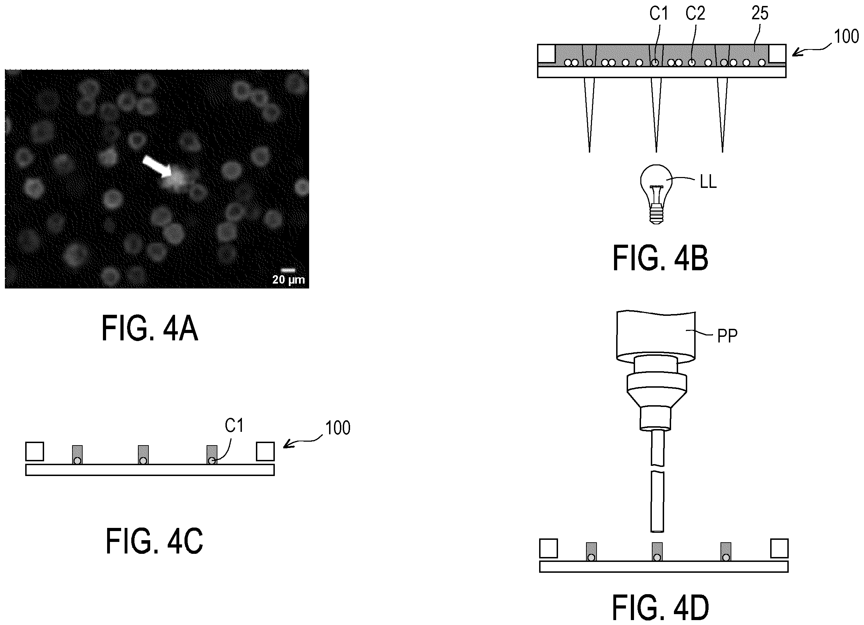

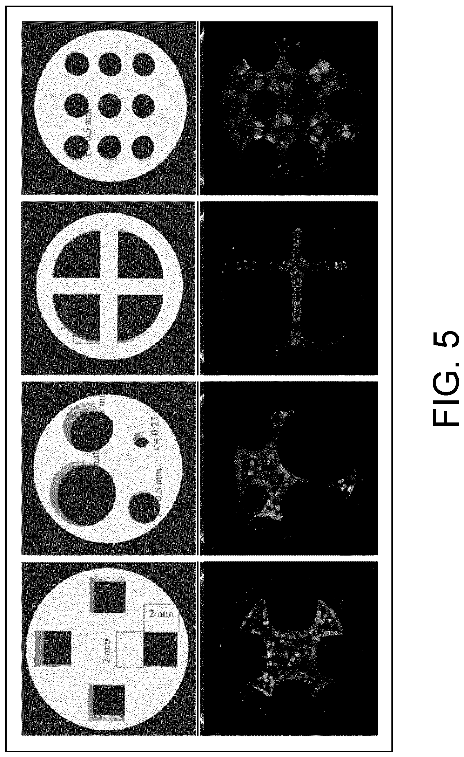

Following Figure 1A to Figure 1D, Figures 4A to 4D are schematic flowcharts of a cell sorting method using a photosensitive hydrogel solution according to an embodiment of the disclosure. Figure 5 is a schematic diagram of a photomask designed and used for irradiating the photosensitive hydrogel solution used in the cell sorting method according to the embodiment of the disclosure.

Referring to Figure 1D and Figure 4A at the same time, the hydrogel solution used is a photosensitive hydrogel solution, which is injected into the cell wells of the array chip in a liquid state, and the cells are spread in a substantially monolayer manner. Then, a fluorescence image analysis is performed to locate the first region with the target cells C1 and the second region with the non-target cells C2. In detail, for example, through a fluorescence image analysis system, for the various fluorescent markers used in the examples, fluorescence scanning is performed on various specific fluorescence excitation bands (meaning the specific fluorescence excitation bands that correspond to specific fluorescent markers), and the images captured in different fluorescence bands are overlaid to confirm the first region and the second region. For example, when the target cells C1 are fetal nucleated red blood cells, and the fluorescent markers are the anti-CD147 antibody with a first fluorescent substance, the anti-CD71 antibody with a second fluorescent substance, and DAPI, the first region is the region where all three, the fluorescence after the first fluorescent substance is excited, the fluorescence after the second fluorescent substance is excited, and the fluorescence after DAPI is excited, overlap at the same time (triple fluorescence overlap region), and the remaining region is defined as the second region. That is to say, the positions of the target cells C1 are determined based on the overlap result of the fluorescence scanning, and the range thereof is defined as the first region. When the target cells C1 are circulating tumor cells, and the fluorescent markers are the anti-EpCAM antibody with the first fluorescent substance, the anti-CD45 antibody with the second fluorescent substance, and the Hoechst 33342, the first region is the region where the excited fluorescence of the first fluorescent substance overlaps with the excited fluorescence of the Hoechst 33342, and does not overlap with the excited fluorescence of the second fluorescent substance, and the other remaining regions are the second regions.

Next, referring to Figure 4B and Figure 5, the first region is irradiated with ultraviolet light to cure the mixture solution 25 in the first region, so as to fix the target cells C1. To be specific, the method of irradiating light LL (e.g. ultraviolet light) with a specific wavelength to the first region to cure the mixture solution 25 in the first region and fix the target cells C1 is, for example: a photomask is designed according to the positions of the first region and the second region (as shown in Figure 5, openings of the photomask correspond to the first region). Next, the photomask is placed above the cell wells 132 of the array chip 100 and irradiated with ultraviolet light with a wavelength of 405 nm for 15 seconds to allow the ultraviolet light to penetrate through the openings of the photomask to irradiate the first region, such that the mixture solution 25 in the first region is cured due to exposure. At this time, since the second region is shielded by the photomask and is not exposed to ultraviolet rays, the mixture solution 25 located in the second region continues to maintain a liquid state.

Next, referring to Figures 4C and Figure 4D, the uncured mixture solution 25 located in the second region is removed. Next, the fixed target cells C1 are aspirated from the first region by the automatic aspirator PP to complete the screening and isolation of the target cells C1. The cell sorting using the photosensitive hydrogel solution according to the embodiment of the disclosure is basically completed herein.

When the hydrogel solution used is a photosensitive hydrogel solution, in addition to what is described in the foregoing embodiments, the purpose of cell sorting can also be achieved by curing different regions according to other embodiments. In other embodiments of the cell sorting method, the method of curing the hydrogel solution and selecting target cells may also include, for example, the following steps: a fluorescence image analysis is performed to locate the first region with target cells and the second region with non-target cells. Next, ultraviolet light is irradiated to the second region to cure the mixture solution located in the second region to fix the non-target cells. Then, the uncured mixture solution is aspirated from the first region using an aspirator so as to obtain the target cells.

In detail, the method of irradiating the second region with ultraviolet light to cure the mixture solution located in the second region and fix the non-target cells is, for example: a photomask is designed according to the positions of the first region and the second region, wherein openings of the photomask correspond to the second region. Next, the photomask is placed above the cell wells of the array chip and irradiated with UV light with a wavelength of 405 nm for 15 seconds to allow the UV light to penetrate through the openings of the photomask to irradiate the second region, such that the mixture solution in the second region is cured due to exposure. At this time, since the first region is shielded by the photomask and is not exposed to ultraviolet rays, the mixture solution located in the first region remains in liquid state. The method of selecting the target cells may be, for example, using an automatic aspirator to aspirate the unfixed target cells from the first region at a flow rate of 20 pl/min, so as to complete the screening and isolation of the target cells.

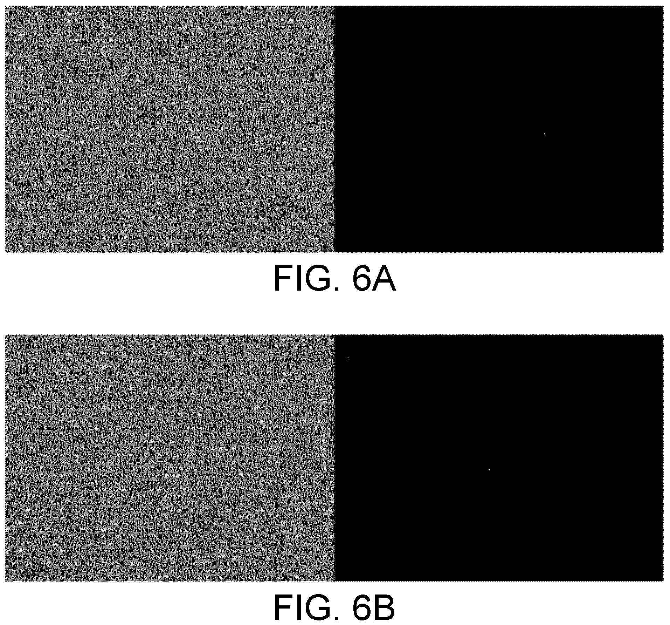

Figure 6A is a cell viability test result after a cell mixture is mixed with the temperature-sensitive hydrogel solution according to an embodiment of the disclosure. Figure 6B is a cell viability test result obtained after extraction and isolation by the cell sorting method according to an embodiment of the disclosure. The left sides of Figures 6A-6B are white light images of cells, which may show all cells under the field of view of microscope. The right sides are the result of propidium iodide (PI) staining, which may stain and mark dead cells. It may be seen from Figure 6A that the temperature-sensitive hydrogel solution used in the disclosure hardly causes cell death, and the average survival rate can reach 96%. It may be seen from Figure 6B that the cell sorting method (such as cooling and curing) of the disclosure combines curing and image analysis to accurately distinguish and screen out the target cells, and accurately aspirates the target cells, respectively. Moderate cell sorting causes almost no target cell death, with an average survival rate of up to 96%.

By using the temperature-sensitive hydrogel solution or the photosensitive hydrogel solution to cure the mixture solution, the target cells and/or non-target cells can be fixed in a container, such that the non-adhesive cells may be sorted. Moreover, by using a temperature-sensitive hydrogel solution or a photosensitive hydrogel solution to cure the mixture solution and the cells therein, the positions of the target cells (or/and non-target cells) in the container are further fixed. Therefore, the target cells can be accurately located during fluorescence image recognition, and specific target cells can be recognized and screened. Also, the accuracy of cell sorting can be further improved by irradiating based on regions. In addition, the method of aspirating cells with an automatic aspirator can reduce the contamination of the solution and the background noise, so higher capture purity can be achieved compared to other isolation methods. Furthermore, due to biocompatibility of the hydrogel solution, and also due to the characteristics such as low light source dose required for photocuring, low shear force of the cell harvesting device, and the absence of chemicals and proteases to release cells and so on, the captured cells can have a very high cell survival rate, such that the sorted cells can be used for subsequent analysis, culture and other applications. In this way, the cell sorting method of this embodiment can be a non-stick cell sorting technology with high efficiency, high purity and easy operation, and is applicable to the field of cell culture and isolation, such as single-cell genomics and proteomics, cellular heterogeneity and so on.

In summary, according to the cell sorting method of the embodiment of the disclosure, by spreading the cell mixture on the bottom of the cell wells in a substantially monolayer manner, the target cells and non-target cells do not overlap in the normal direction of the cell wells. Next, by curing the mixture solution including the hydrogel solution, the target cells and/or non-target cells may be fixed on the bottom of the cell wells. Then, by fluorescence image analysis, the target cells that are bound with the fluorescent markers and emit fluorescence may be located, such that the target cells can be precisely selected and isolated from the cell mixture. In this way, the cell sorting method of this embodiment can improve the accuracy and capture purity of cell sorting, so as to reduce cell damage and achieve the effect of high cell recovery rate.

It will be apparent to those skilled in the art that various modifications and variations may be made to the structure of the disclosure without departing from the scope or spirit of the disclosure. In view of the foregoing, it is intended that the disclosure cover modifications and variations of this disclosure provided they fall within the scope of the following claims and their equivalents.

## Claims

1. A cell sorting method, **characterized by** comprising:
providing a blood sample;
centrifuging the blood sample to obtain a cell mixture having target cells and non-target cells;
adding fluorescent markers to the cell mixture to allow the fluorescent markers to bind with the target cells;
adding a hydrogel solution and mixing the same with the cell mixture to form a mixture solution;
distributing the mixture solution to cell wells of an array chip and spreading the target cells and the non-target cells in the cell mixture on a bottom of the cell wells in a substantially monolayer manner;
curing the mixture solution; and
performing a fluorescence image analysis to select the target cells that are bound with the fluorescent markers and emit fluorescence, and isolating the selected target cells.

2. A cell sorting method according to claim 1, **characterized in that** the hydrogel solution comprises a temperature-sensitive hydrogel solution, and a step of performing the fluorescence image analysis is performed after a step of curing the mixture solution, wherein the step of curing the mixture solution comprises performing a cooling process to cure the mixture solution, fixing positions of the target cells and the non-target cells, and then performing the fluorescence image analysis to locate the positions of the target cells that emit fluorescence so as to select the target cells that are bound with the fluorescent markers and emit fluorescence.

3. The cell sorting method according to claim 2, **characterized in that**, taking a weight of the temperature-sensitive hydrogel solution as 100% by weight, the temperature-sensitive hydrogel solution comprises 3% to 5% by weight of gelatin.

4. The cell sorting method according to claim 1, **characterized in that** the hydrogel solution comprises a photosensitive hydrogel solution, and a step of performing the fluorescence image analysis is performed before a step of curing the mixture solution, wherein the step of performing the fluorescence image analysis comprises:
performing the fluorescence image analysis to locate a first region that emits fluorescence and a second region that does not emit fluorescence, and
wherein the step of curing the mixture solution comprises:
irradiating the first region with ultraviolet light to cure the mixture solution located in the first region, so as to fix the target cells.

5. The cell sorting method according to claim 4, **characterized in that** a step of isolating the selected target cells comprises:
removing the uncured mixture solution located in the second region after irradiating the first region with ultraviolet light; and
aspirating the fixed target cells from the first region using an aspirator.

6. The cell sorting method according to claim 1, **characterized in that** the hydrogel solution comprises a photosensitive hydrogel solution, and a step of performing the fluorescence image analysis is performed before a step of curing the mixture solution, wherein the step of performing the fluorescence image analysis comprises:
performing the fluorescence image analysis to locate a first region that emits fluorescence and a second region that does not emit fluorescence, and
wherein the step of curing the mixture solution comprises:
irradiating the second region with ultraviolet light to cure the mixture solution located in the second region.

7. The cell sorting method according to claim 6, **characterized in that** a step of isolating the selected target cells comprises:
aspirating the uncured mixture solution from the first region by an aspirator to obtain the target cells after irradiating ultraviolet light to the second region.

8. The cell sorting method according to claim 4 or claim 6, **characterized in that** taking a weight of the photosensitive hydrogel solution as 100% by weight, the photosensitive hydrogel solution comprises:
10% to 20% by weight of polyethylene glycol diacrylate; and
0.1% to 1% by weight of photoinitiator.

9. The cell sorting method according to claim 1, **characterized in that** cells in the cell mixture are non-adhesive cells, comprising mononuclear lymphocytes, circulating tumor cells, fetal nucleated red blood cells, platelets, or a combination thereof.

10. A cell sorting method according to claim 1, **characterized in that** the target cells are fetal nucleated red blood cells, the fluorescent markers comprise an anti-CD147 antibody with a first fluorescent substance and an anti-CD71 antibody with a second fluorescent substance, and a step of adding the fluorescent markers to the cell mixture comprises:
adding the anti-CD147 antibody and the anti-CD71 antibody to allow the anti-CD147 antibody and the anti-CD71 antibody to bind with the target cells, wherein the first fluorescent substance is different from the second fluorescent substance.

11. The cell sorting method according to claim 10, **characterized in that** the fluorescent markers further comprise 4',6-diamidino-2-phenylindole for binding with the target cells.

## Amended claims

### Amended claims under Art. 19.1 PCT

1. A cell sorting method, **characterized by** comprising:
providing a blood sample;
centrifuging the blood sample to obtain a cell mixture having target cells and non-target cells;
adding fluorescent markers to the cell mixture to allow the fluorescent markers to bind with the target cells;
adding a hydrogel solution and mixing the same with the cell mixture to form a mixture solution;
distributing the mixture solution to cell wells of an array chip and spreading the target cells and the non-target cells in the cell mixture on a bottom of the cell wells in a substantially monolayer manner;
curing the mixture solution, and at least one of the target cells and the non-target cells embedded within the curried mixture solution; and
performing a fluorescence image analysis to select the target cells that are bound with the fluorescent markers and emit fluorescence, and isolating the selected target cells.

2. A cell sorting method according to claim 1, **characterized in that** the hydrogel solution comprises a temperature-sensitive hydrogel solution, and a step of performing the fluorescence image analysis is performed after a step of curing the mixture solution, wherein the step of curing the mixture solution comprises performing a cooling process to cure the mixture solution, fixing positions of the target cells and the non-target cells, and then performing the fluorescence image analysis to locate the positions of the target cells that emit fluorescence so as to select the target cells that are bound with the fluorescent markers and emit fluorescence.

3. The cell sorting method according to claim 2, **characterized in that**, taking a weight of the temperature-sensitive hydrogel solution as 100% by weight, the temperature-sensitive hydrogel solution comprises 3% to 5% by weight of gelatin.

4. The cell sorting method according to claim 1, **characterized in that** the hydrogel solution comprises a photosensitive hydrogel solution, and a step of performing the fluorescence image analysis is performed before a step of curing the mixture solution, wherein the step of performing the fluorescence image analysis comprises:
performing the fluorescence image analysis to locate a first region that emits fluorescence and a second region that does not emit fluorescence, and
wherein the step of curing the mixture solution comprises:
irradiating the first region with ultraviolet light to cure the mixture solution located in the first region, so as to fix the target cells.

5. The cell sorting method according to claim 4, **characterized in that** a step of isolating the selected target cells comprises:
removing the uncured mixture solution located in the second region after irradiating the first region with ultraviolet light; and
aspirating the fixed target cells from the first region using an aspirator.

6. The cell sorting method according to claim 1, **characterized in that** the hydrogel solution comprises a photosensitive hydrogel solution, and a step of performing the fluorescence image analysis is performed before a step of curing the mixture solution, wherein the step of performing the fluorescence image analysis comprises:
performing the fluorescence image analysis to locate a first region that emits fluorescence and a second region that does not emit fluorescence, and
wherein the step of curing the mixture solution comprises:
irradiating the second region with ultraviolet light to cure the mixture solution located in the second region.

7. The cell sorting method according to claim 6, **characterized in that** a step of isolating the selected target cells comprises:
aspirating the uncured mixture solution from the first region by an aspirator to obtain the target cells after irradiating ultraviolet light to the second region.

8. The cell sorting method according to claim 4 or claim 6, **characterized in that** taking a weight of the photosensitive hydrogel solution as 100% by weight, the photosensitive hydrogel solution comprises:
10% to 20% by weight of polyethylene glycol diacrylate; and
0.1% to 1% by weight of photoinitiator.

9. The cell sorting method according to claim 1, **characterized in that** cells in the cell mixture are non-adhesive cells, comprising mononuclear lymphocytes, circulating tumor cells, fetal nucleated red blood cells, platelets, or a combination thereof.

10. A cell sorting method according to claim 1, **characterized in that** the target cells are fetal nucleated red blood cells, the fluorescent markers comprise an anti-CD147 antibody with a first fluorescent substance and an anti-CD71 antibody with a second fluorescent substance, and a step of adding the fluorescent markers to the cell mixture comprises:
adding the anti-CD147 antibody and the anti-CD71 antibody to allow the anti-CD147 antibody and the anti-CD71 antibody to bind with the target cells, wherein the first fluorescent substance is different from the second fluorescent substance.

11. The cell sorting method according to claim 10, **characterized in that** the fluorescent markers further comprise 4',6-diamidino-2-phenylindole for binding with the target cells.
